# EUROPÄISCHE PATENTSCHRIFT

(11) **EP 2 048 969 B1**
(45) Veröffentlichungstag und Bekanntmachung des Hinweises auf die Patenterteilung: **10.12.2014**
(21) Anmeldenummer: 07788206.6
(22) Anmeldetag: 03.08.2007
(51) Int. Cl.: A23J 1/20, A23L 1/305, A61K 38/01, A61P 3/10

(54) **FRAKTIONEN AUS MOLKEPERMEAT UND DEREN VERWENDUNG ZUR PRÄVENTION UND THERAPIE DES TYP-2 DIABETES UND DES METABOLISCHEN SYNDROMS**
FRACTIONS OF WHEY PERMEATE AND ITS USE FOR THE PREVENTION AND THERAPY OF TYPE 2 DIABETES AND THE METABOLIC SYNDROME
FRACTIONS DE PERMÉAT DE LACTOSÉRUM ET LEUR UTILISATION POUR LA PRÉVENTION ET LA THÉRAPIE DU DIABÈTE DE TYPE 2 ET DU SYNDROME MÉTABOLIQUE

(30) Priorität: 03.08.2006 DE 102006036285
(43) Veröffentlichungstag der Anmeldung: 22.04.2009
(73) Patentinhaber: S.U.K. Beteiligungs GmbH, 1010 Wien (AT)
(72) Erfinder: KRAUSKOPF, Jobst, 29576 Barum/Krs. Uelzen (DE)
(74) Vertreter: Hoffmann Eitle
(86) Internationale Anmeldenummer: PCT/EP2007/058073
(87) Internationale Veröffentlichungsnummer: WO 2008/015275

(56) Entgegenhaltungen:
- EP-A- 0 629 350
- WO-A-01/37850
- WO-A-2005/004990
- MACLEOD A ET AL: "SEPARATION OF BETA-LACTOGLOBULIN VARIANTS A AND B FROM CHEESE WHEY BY BIOSPECIFIC SUBUNIT EXCHANGE AFFINITY CHROMATOGRAPHY" MILCHWISSENSCHAFT, VV GMBH VOLKSWIRTSCHAFTLICHER VERLAG. MUNCHEN, DE, Bd. 50, Nr. 8, 1. September 1995 (1995-09-01), Seiten 440-444, XP000533381 ISSN: 0026-3788
- MUTHUKUMARAN S ET AL: "Mechanisms for the ultrasonic enhancement of dairy whey ultrafiltration" JOURNAL OF MEMBRANE SCIENCE, ELSEVIER SCIENTIFIC PUBL.COMPANY. AMSTERDAM, NL, Bd. 258, Nr. 1-2, 1. August 2005 (2005-08-01), Seiten 106-114, XP004936313 ISSN: 0376-7388
- DATABASE EPODOC EUROPEAN PATENT OFFICE, THE HAGUE, NL; XP002461114 & RU 2005 128653 A (OOO MEDBIOFARM BIOTEKH [RU]) 20. März 2007 (2007-03-20)

## Beschreibung

### Technisches Gebiet

Die vorliegende Erfindung betrifft die pharmazeutische und die Nahrungsmittelindustrie. In der Nahrungsmittelindustrie ist insbesondere der Bereich von Nahrungsergänzungsmitteln (oder "functional food") von Bedeutung. Insbesondere ist vorliegend an die Prävention und Behandlung der Insulinresistenz und Glucoseintoleranz durch Verwendung von Fraktionen aus Molke gedacht.

Insulinresistenz und Glucoseintoleranz treten bei Personen auf, die gefährdet sind, das Metabolische Syndrom oder einen Typ 2-Diabetes zu entwickeln, oder sie sind Symptome der bereits diagnostizierten Erkrankungen.

Nach WHO-Kriterien sind 63 % aller Männer und 55 % aller Frauen in den industrialisierten Ländern übergewichtig. Ursache dieser bedenklichen Entwicklung ist die kalorische Überernährung mit Kohlenhydraten und Fetten bei weiterer Absenkung der täglichen Energieabgabe. Schon sehr kleine Differenzen in der täglichen Energiebilanz erzeugen über die Jahre einen großen kumulativen Effekt auf die Körpermasse. So führt eine täglich 0,3 % über dem Kalorienverbrauch liegende Kalorienzufuhr zu einer Gewichtszunahme von 9.1 kg bei 25 bis 55 Jahre alten Amerikanern. Übergewichtigen entwickeln häufig das so genannte Metabolische Syndrom (Reaven GM, Curr. Opin. Lipidol., 1997, 8 (1), S. 23-27). Hierunter versteht man das gemeinsame Vorliegen von Übergewicht (Fettsucht),

Bluthochdruck, erhöhtem Insulinspiegel und Fettstoffwechselstörung. In der Fachliteratur wird das Metabolische Syndrom auch als 1. Frühsyndrom oder "Prädiabetes" bezeichnet (Pschyrembel, Klinisches Wörterbuch, 257 Aufl. (1994), S. 321).

Unter dem Begriff "Syndrom" versteht der Fachmann einen Komplex aus Symptomen, d.h. eine Gruppe von Krankheitszeichen, die für ein bestimmtes Krankheitsbild mit meist uneinheitlicher oder unbekannter Ätiologie bzw. Pathogenese charakteristisch sind.

Mit weiterer Verschlechterung des Metabolischen Syndroms entwickelt sich häufig ein Typ 2-Diabetes. Hervorstechendes Merkmal hierfür ist die Erhöhung des Blutzuckers. Des Weiteren begünstigen diese Erkrankungen ein erhöhtes Risiko von Folgeerkrankungen, beispielsweise Arterienverkalkung. Weitere Gesundheitsrisiken die durch die pathologischen Veränderungen beim Diabetes bedingt sind, sind Augenleiden, Herz- und Gefäßerkrankungen, Schlaganfall, Herzinfarkt, Nierenerkrankungen etc.

In den vergangenen Jahrzehnten hat die Häufigkeit des Typ 2-Diabetes erheblich zugenommen. Das Statistische Bundesamt schätzt, dass derzeit rund vier Millionen Menschen in Deutschland betroffen sind. Andere Studien gehen jedoch von erheblich höheren Zahlen aus. Nach Schätzungen der Deutschen Diabetes Gesellschaft, wird 2006 jeder 10 Einwohner Deutschlands vom Typ 2-Diabetes betroffen sein. Bei übergewichtigen Diabetikern sind zumeist auch die Blutfettspiegel (Cholesterin, Triglyzeride) und der Blutdruck erhöht.

Im Gegensatz zum Typ 1-Diabetes wird beim Typ 2-Diabetes zwar Insulin produziert. Dieses kann vom Körper jedoch nicht mehr richtig verwertet werden. Insulin wird in der Bauchspeicheldrüse gebildet und sorgt dafür, dass Glucose aus der Nahrung in die Zellen gelangt. Wenn dem Körper durch die Nahrung Glucose zugeführt wird und der Blutzuckerspiegel im Blut ansteigt, wird vermehrt Insulin in das Blut abgegeben, um die Glucose in die Zellen zu bef ördern - der Blutzuckerspiegel sinkt dadurch wieder. Wenn der Körper nicht mehr richtig auf das Insulin reagiert, werden die Zellen insulinresistent. Laut Roche Lexikon Medizin, 4. Auflage, Urban & Fischer Verlag, 1999, versteht man unter Insulinresistenz die starke Minderung oder das Ausbleiben der therapeutischen Insulinwirkung. Für das Entstehen einer Insulinresistenz gibt es drei Erklärungen. Erstens können IgG-Antikörper die biologische Wirksamkeit des Insulins hemmen und dadurch den Bedarf auf über 100 IE (Internationale Einheiten) täglich erhöhen. Zweitens kann es zu einer erhöhten enzymatischen Insulinspaltung kommen, oder drittens die Bindung des Insulins an seine Rezeptoren herabgesetzt sein. Als Folge gelangt nicht mehr ausreichend Energie in die Zellen, während der Blutzuckerspiegel hoch bleibt. Das führt dazu, dass die Bauchspeicheldrüse noch mehr Insulin ausschüttet, um den Blutzuckerspiegel zu senken. Durch die ständige Überproduktion von Insulin kommt es zu einer Erschöpfung der Insulin-produzierenden Beta-Zellen in der Bauchspeicheldrüse und schließlich zum Insulinmangel.

Heutzutage werden Typ 2-Diabetiker häufig mit oralen Antidiabetika behandelt. Hierzu zählen bspw. Alpha-Glukosidasehemmer, Biguanide. Glitazone, Glinide, oder Sulfonylharnstoffe.

Neben oralen Antidiabetika kann es notwendig werden, dass dem Patient Insulin zugeführt wird. Hierbei werden individualisierte Therapieformen durchgeführt, die auf die Bedürfnisse der Patienten zugeschnitten sind.

Die Behandlung des Metabolischen Syndroms beruht im Wesentlichen darauf, dass die Patienten durch eine Ernährungsumstellung und vermehrte Bewegung eine Gewichtsabnahme erreichen. Oft sind jedoch zusätzlich Medikamente zur Blutdruck-, Blutzucker- und Blutfettsenkung notwendig. Schätzungen hinsichtlich der Kosten, die jährlich in den USA durch direkte Aufwendungen und indirekte Kosten durch den Produktivitätsverlust durch die oben genannten Behandlungsformen anfallen, belaufen sich auf insgesamt 98 Milliarden Dollar - bei steigender Tendenz.

Im Fachgebiet der Verwendung von Molke zur Behandlung von Diabetes beschreibt WO 01/37850 ein Milchproteinhydrolysat, insbesondere die Verwendung des darin enthaltenen Caseinoglycomacropeptid (CGMP) zur Behandlung von Diabetes. CGMP hat ein Molekulargewicht von circa 5000 Dalton und wird unter den Peptiden der erfindungsgemäß bereitgestellten Molkeanteile nicht gefunden, wie vorbereitende Untersuchungen im Rahmen dieser Anmeldung gezeigt haben. Andere isolierte Bestandteile werden nicht beschrieben.

WO 2005/004990 beschreibt die Verwendung von Molkepermeat, bevorzugt Süßmolkepermeat, noch bevorzugter von hydrolysiertem oder teilhydrolysiertem Süßmolkepermeat zur Herstellung einer pharmazeutischen Zusammensetzung zur Prophylaxe oder Behandlung von Symptomen des Metabolischen Syndroms, des Diabetes Typ-2 und Folgeerkrankungen bei Säugern.

### Ziele der Erfindung

Deshalb ist es notwendig und ein Ziel der Anmeldung, weitere Mittel und Wege zur Prävention und Behandlung der Glucoseintoleranz und der Insulinresistenz zur Verfügung zu stellen, um so das Metabolische Syndroms oder den Typ 2-Diabetes zu verhindern, bzw. diese Erkrankungen zu behandeln. Des Weiteren werden Mittel zur Verhinderung oder Verzögerung der Verschlechterung der Symptome sowie zu deren Linderung oder Heilung benötigt.

Intensive Studien der Anmelderin haben dazu geführt, dass Fraktionen mit der gewünschten Wirkung aus Molkepermeat, bevorzugt aus Süßmolkepermeat, noch bevorzugter aus lactosereduziertem und/oder hydrolysiertem bzw. teilhydrolysiertem Süßmolkepermeat identifiziert wurden, welche die vorteilhaften Wirkungen im Rahmen der Prävention oder Behandlung des Metabolischen Syndroms, des Typ 2-Diabetes und dadurch begünstigter Folgeerkrankungen haben.

Unter dem Begriff "Prävention" wird folgend verstanden, dass das Entstehen eines Symptoms oder einer Erkrankung verhindert wird.

Unter dem Begriff "Behandlung" wird nachfolgend jede Form einer Behandlung zur Verbesserung der Krankheitssymptome, zur Verzögerung der Progression der Krankheit, zur Regression der Krankheit oder zur Linderung der Krankheitssymptome verstanden.

Unter dem Begriff "pharmazeutische Zusammensetzung" wird jede dem Fachmann bekannte Form verstanden. Bevorzugt sind oral verabreichbare Zusammensetzungen.

Unter dem Begriff "Nahrungsergänzungsmittel" wird jedes Nahrungsmittel verstanden, dem die erfindungsgemäßen Molkebestandteile zugesetzt werden können, z.B. Säfte, Sirups, Mineralwässer, Milchprodukte, Müsliriegel, Gewürzmischungen, Fertiggerichte (z.B. Suppen, tiefgekühlte Produkte etc.), Süßigkeiten etc.

Unter dem Begriff "isolierte Fraktion" wird erfindungsgemäß jeder Bestandteil der Molke verstanden, der durch (bio-) chemische oder physikalische Methoden so aufbereitet wurde, dass er abgetrennt und/oder isoliert bereitgestellt werden kann. Beispiele für Trennverfahren sind z.B. Zentrifugieren, Filtrieren oder chromatographische Auftrennungsverfahren. Bevorzugt sind chromatographische Auftrennungsverfahren. Bevorzugte Fraktionen sind Peptidfraktionen. Diese können ein oder mehrere Peptide umfassen. Die Peptide können glykosyliert sein, oder sonstige dem Fachmann bekannte Substitutionen haben. Wenn nachfolgend von Fraktionen aus Molkepermeat gesprochen wird, sind isolierte Fraktionen gemeint.

Eine Aufgabe der vorliegenden Erfindung liegt darin, Fraktionen aus Molkepermeat bereit zu stellen, die zur Prävention oder Behandlung der oben genannten Symptome, insbesondere der Glukoseintoleranz oder Insulinresistenz verwendet werden können.

In einer Ausführungsform der Erfindung wird mit Hilfe einer oder mehrerer dieser Molke-Fraktionen dem Entstehen des Metabolischen Syndroms oder des Typ 2-Diabetes entgegengewirkt oder deren Behandlung ermöglicht. Ebenfalls in den Bereich der vorliegenden Erfindung eingeschlossen sind Behandlung, Linderung bzw. Prävention von Folgeerkrankungen des Diabetes Typ 2, wie Arteriosklerose, Glaukome, Xanthome, etc.

Die erfindungsgemäßen Fraktionen umfassen Peptide mit einer Masse von 100 bis 3500 Dalton. Weitere bevorzugte Größenbereiche sind 200 bis 3000 Dalton; 200 bis 2800 Dalton; 200 bis 2500 Dalton; 200, 300 oder 400 bis 2000 Dalton; 200 bis 1700 Dalton; 200 bis 1600 Dalton oder 200 bis 1500 Dalton. Der am meisten bevorzugte Bereich umfasst Peptide der Größe 500 bis 2800. Insbesondere umfasst der am meisten bevorzugte Bereich hydrophobe Peptide von 500 bis 3000 Dalton, und hydrophile Peptide von 500 bis 2500 Dalton.

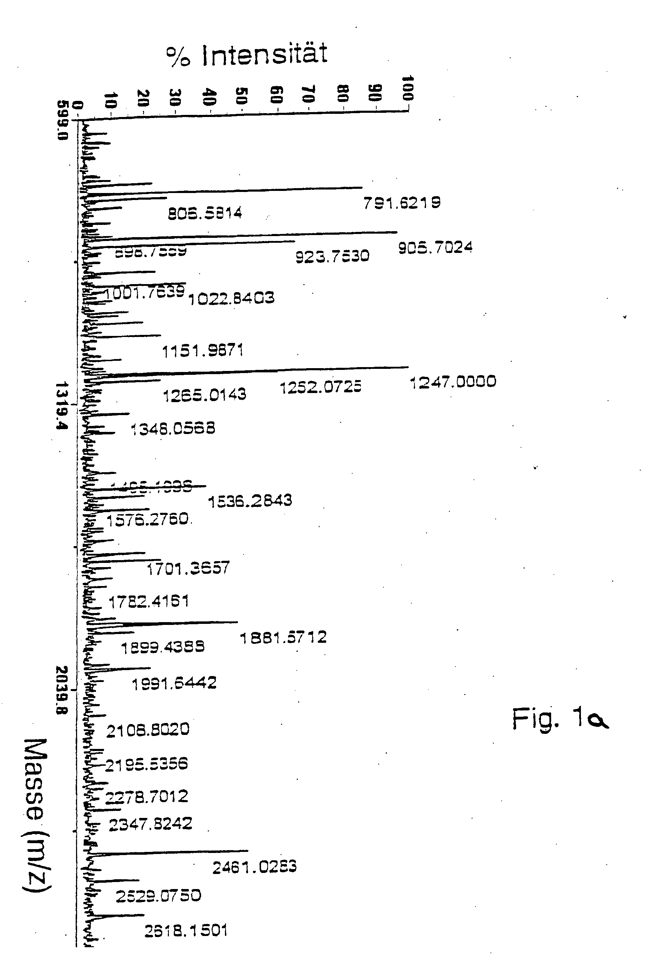

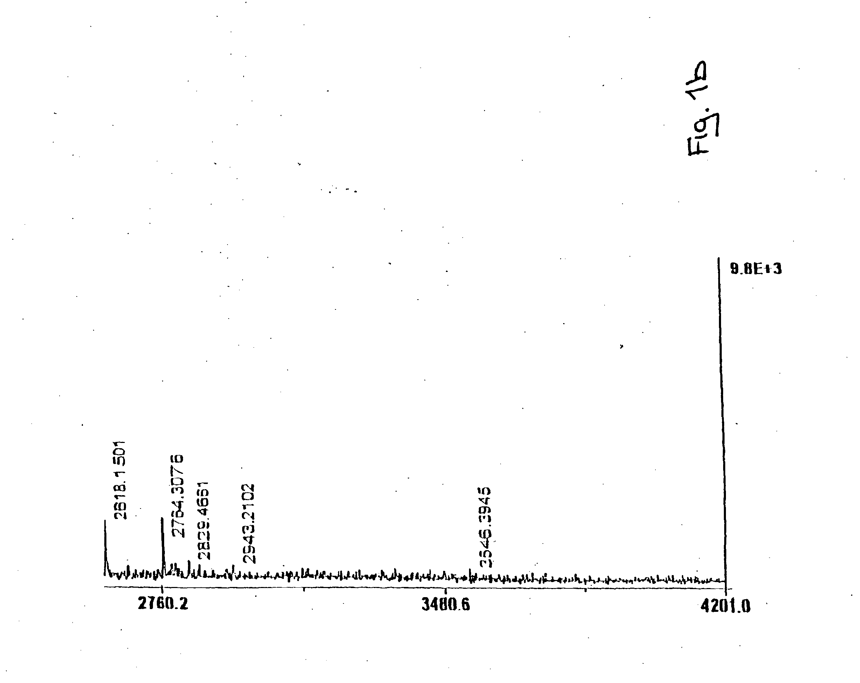

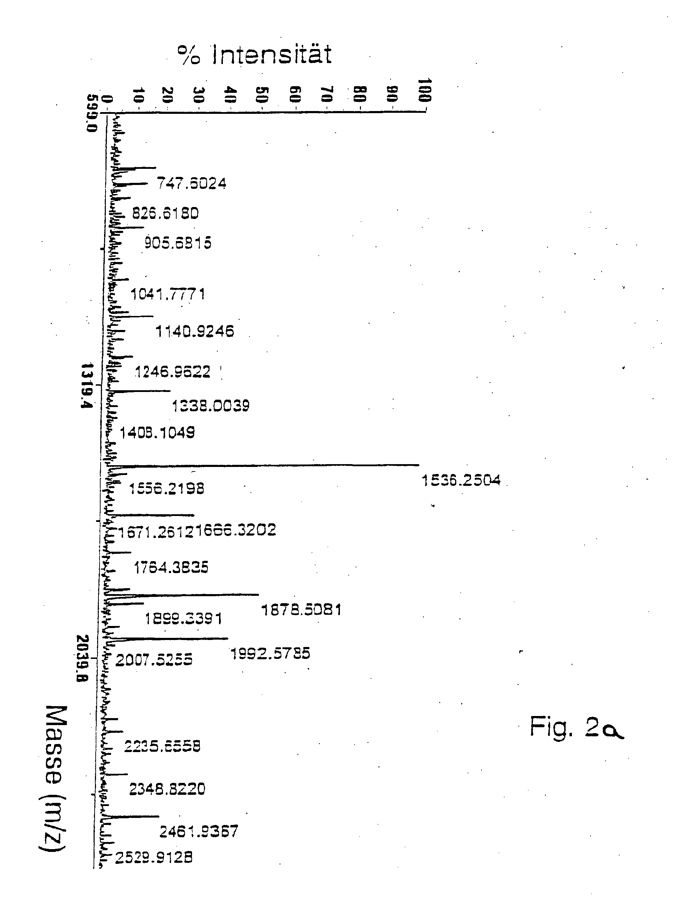

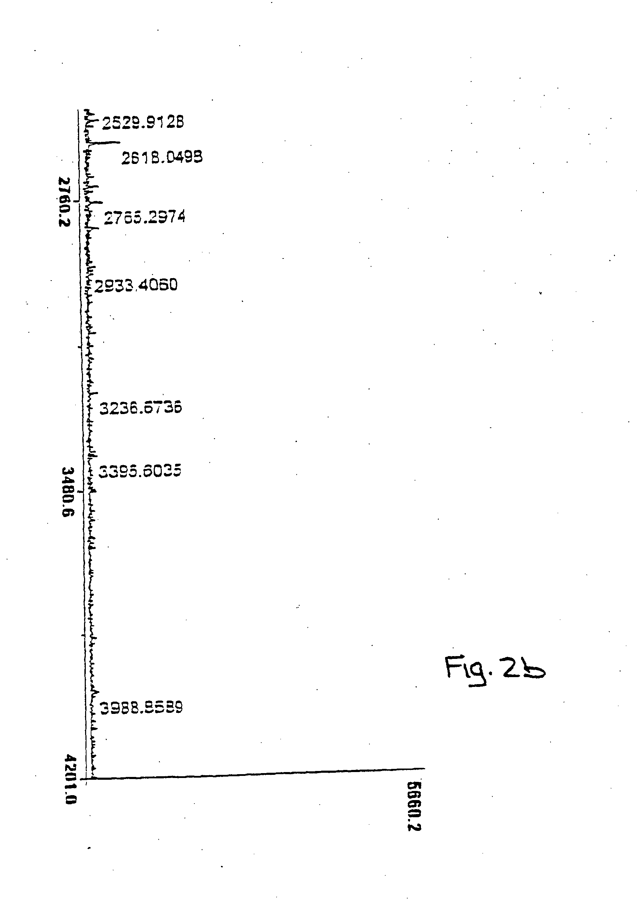

Nach einer bevorzugten Ausführungsform der vorliegenden Erfindung sind die wirksamen Molke-Fraktionen unter den hydrophoben oder hydrophilen Peptiden zu finden. Die hydrophoben Peptide und Fraktionen hydrophober Peptide sind bevorzugt. Besonders bevorzugt sind Fraktionen, die Peptide mit den in den Figuren 1 und 2 gezeigten Peptid-Massen umfassen.

Dass wirksame Fraktionen des Molkepermeat insbesondere hydrophobe Peptide mit den oben genannten Massen umfassen, wurde durch vergleichende Untersuchungen der Wirkung verschiedener Bestandteile der Molke in biochemischen Modellsystemen mit besonderer Relevanz zum diabetischen Geschehen untersucht.

Die vorliegende Erfindung stellt Fraktionen aus Molkepermeat, sowie die Verwendung der Fraktionen bereit. Zusammengefasst werden bereitgestellt:
(1) Isolierte Fraktion aus Süßmolkepermeat, umfassend ein oder mehrere Polypeptide mit einer Masse von 100 bis 3500 Dalton, vorzugsweise 200 bis 3000 Dalton.
(2) Isolierte Fraktion aus Süßmolkepermeat gemäß (1), umfassend hydrophobe Polypeptide mit einer Masse von 500 bis 3000 Dalton.
(3) Isolierte Fraktion aus Süßmolkepermeat gemäß (1), umfassend ein oder mehrere hydrophile Polypeptide mit einer Masse von 500 bis 2500 Dalton.
(4) Isolierte Fraktion aus Süßmolkepermeat gemäß (1) bis (3), dadurch gekennzeichnet, dass das Süßmolkepermeat hydrolysiert oder teilhydrolysiert ist.
(5) Pharmazeutische Zusammensetzung umfassend eine isolierte Fraktion aus Süßmolkepermeat gemäß (1) bis (4).
(6) Isolierte Fraktion aus Süßmolkepermeat gemäß (1) bis (4) zur Verwendung als Medikament.
(7) Isolierte Fraktion aus Süßmolkepermeat gemäß (1) bis (4) zur Verwendung bei der Prävention oder Behandlung von Typ-2 Diabetes, Metabolischem Syndrom oder Folgeerkrankungen davon.
(8) Verwendung mindestens einer isolierten Fraktion aus Süßmolkepermeat gemäß (1) bis (4) zur Herstellung eines Medikaments zur Prävention oder Behandlung von Typ-2 Diabetes, Metabolischem Syndrom oder Folgeerkrankungen davon.
(9) Nahrungsergängzungsmittel, umfassend mindestens eine der isolierten Fraktionen aus Süßmolkepermeat gemäß (1) bis (4).

### Beschrieben hierin sind:

1) Verfahren zur Herstellung von Fraktionen aus Molkepermeat, gekennzeichnet durch die Schritte:
   a) Suspendieren von Molkepermeat,
   b) Ultraschallbehandlung des suspendierten Molkepermeats
   c) Zentrifugieren der Suspension aus Schritt (b)
   d) Chromatographische Auftrennung der in Schritt (c) gewonnenen Überstände,
   e) Eluieren der aufgetrennten Fraktionen des Molkepermeats.
2) Verfahren gemäß (1), worin die chromatographische Auftrennung mittels HPLC durchgeführt wird.
3) Verfahren gemäß (1) oder (2), worin die Auftrennung mit einer C18-Kieselgel-Chromatographie-Säule durchgeführt wird.
4) Verfahren gemäß (1) bis (3), wobei das Molkepermeat Süßmolkepermeat, ist.
5) Verfahren gemäß (4), wobei das Süßmolkepermeat hydrolysiert oder teilhydrolysiert ist.
6) Isolierte Fraktion aus Molkepermeat erhältlich durch das Verfahren nach (1) bis (5).
7) Isolierte Fraktion aus Molkepermeat, gemäß (6), umfassend ein oder mehrere Polypeptide mit einer Masse von <5000 Dalton.
8) Isolierte Fraktion aus Molkepermeat gemäß (6) oder (7), dadurch gekennzeichnet, dass sie Peptide umfasst, die in Figur 1 bzw. 2 gezeigt sind.
9) Isolierte Fraktion aus Molkepermeat gemäß (6) oder (7), oder wie in Figur 1 gezeigt, worin mindestens eines der Peptide hydrophob ist.
10) Isolierte Fraktion aus Molkepermeat gemäß (6) oder (7), oder wie in Figur 1 gezeigt, worin alle Polypeptide hydrophob sind.
11) Pharmazeutische Zusammensetzung umfassend mindestens eine isolierte Fraktion aus Molkepermeat gemäß (6) bis (11).
12) Verwendung mindestens einer isolierten Fraktion aus Molkepermeat gemäß (6) bis (11) als Medikament.
13) Verwendung mindestens einer isolierten Fraktion aus Molkepermeat gemäß (6) bis (11), zur Herstellung eines Medikaments zur Prävention oder Behandlung von Typ-2 Diabetes, Metabolischem Syndrom oder Folgeerkrankungen davon.
14) Nahrungsergänzungsmittel, umfassend mindestens eine der isolierten Fraktionen aus Molkepermeat gemäß irgendeinem der aus (6) bis (11).

### Beschreibung der Figuren

Figuren 1 und 2 zeigen Massenspektrogramme der hydrophoben bzw. hydrophilen Proteine/Peptide des Süßmolkepermeat nach der Auftrennung im erfindungsgsmäßen Verfahren. In den Figuren sind die Massen der von den Fraktionen umfassten Peptide auf der Abszisse gezeigt, während die Intensität der jeweiligen Peptide auf der Ordinate angegeben ist.

### Detaillierte Beschreibung der Erfindung

Erfindungsgemäß werden isolierte Fraktionen aus Molkepermeat, zur Verfügung gestellt, die bevorzugt Peptide der oben beschriebenen Massen umfassen. Das erfindungsgemäß verwendete Molkepermeat wird bevorzugt aus Süßmolke gewonnen.

- Die Aufreinigung der Fraktionen aus Molkepermeat erwies sich als äußerst schwierig. Um die Schwierigkeiten zu überwinden, wurde ein spezielles HPLC-Aufreinigungsverfahren entwickelt, das die Bereitstellung der erfindungsgemäßen Fraktionen in hinreichender Ausbeute und Reinheit ermöglichte. Dieses Verfahren zur Aufreinigung von Molkepeptiden ist hierin beschrieben. Durch das Verfahren konnten erstmals die vom Anspruch umfassten, isolierten Fraktionen hergestellt werden. Massenspektrometrische Ergebnisse der erhaltenen hydrophoben bzw. hydrophilen Molkepeptidfraktionen werden in den Figuren 1 bzw. 2 gezeigt.

Das Verfahren umfasst die in den Ansprüchen angegebene Abfolge der notwendigen Schritte.

Notwendige Schritte sind die nachfolgenden Schritte (a) bis (e) :
a) Suspendieren von Molkepermeat,
b) Ultraschallbehandlung des suspendierten Molkepermeats
c) Zentrifugieren der Suspension aus Schritt (b)
d) Chromatographische Auf trennung der in Schritt (c) gewonnenen Überstände,
e) Eluieren der aufgetrennten Fraktionen des Molkepermeats.

Schritt (a) betrifft die Suspension von gepulvertem/nicht gepulvertem Molkepermeat in einem Medium. Beispiele für mögliche Medien sind Wasser, Phosphat-gepufferte Salzlösung (PBS), oder angesäuerte wässrige Lösungen.

Besonders bevorzugt sind angesäuerte wässrige Lösungen, insbesondere 0,05 bis 1,0 Vol.-% Lösungen von Trifluoressigsäure, Salzsäure oder anderer organischer oder anorganischer Lösungen in Wasser (z.B. bidestilliertes Wasser, Wasser zur Injektion oder Merck-Wasser (Fluka Kat.Nr. 91699)). Am meisten bevorzugt sind 0,05 bis 0, 25, insbesondere 0,1 Vol.-% Lösungen von Trifluoressigsäure in Wasser.

Die Suspensionen werden bei Temperaturen von 0 bis 50°C hergestellt, bevorzugt bei Temperaturen von 4 bis 37°C und besonders bevorzugt bei ca. 25°C. Die Suspendierung kann durch Rühren erleichtert werden. Bevorzugt werden die in einem Becherglas vermischten Reagenzien auf einem Magnetrührer gemischt. Dieser Vorgang wird durchgeführt, bis kein Bodensatz mehr zu erkennen ist. Der Mischvorgang ist abhängig von der Konzentration des verwendeten Molkepermeats und kann einige Minuten bis mehrere Stunden dauern. Bei einer einprozentigen Suspension Molkepermeat in Lösungsmittel dauert dieser Vorgang ca. 10 bis 60 Minuten, jedoch reichen 30 Minuten bei 25°C meistens aus.

Schritt (b) betrifft die Ultraschallbehandlung der erhaltenen Suspension. Hierzu wird die erhaltene Suspension in einem Gefäß in ein herkömmliches Ultraschallgerät gestellt. Bevorzugt steht oder schwimmt das Gefäß in einem Ultraschallbad und wird für einige Minuten, bevorzugt 1 bis 20 Minuten behandelt. Die Behandlung für 1 bis 5 Minuten, z.B. 2, 3 oder 4 Minuten ist in der Regel bei maximaler Beschallung (z.B. 60W) ausreichend. Dem Fachmann ist bekannt, dass die Dauer der Behandlung der Suspension von Faktoren, wie der Leistung des Ultraschallgeräts, der Temperatur, dem Volumen etc abhängt.

Im Schritt (c), der das Zentrifugieren der erhaltenen Suspension betrifft, werden nicht oder schwer lösliche Bestandteile von den löslichen getrennt. Es kann jede handelsübliche Zentrifuge verwendet werden, vorausgesetzt sie erlaubt eine Umdrehungszahl von 5.000 bis 20.000 x g. Bevorzugt werden Umdrehungszahlen von 10.000 bis 17.000 x g, besonders bevorzugt sind Umdrehungszahlen von 12.500 bis 15.000 x g, z.B. 14.000 x g.

Die Dauer dieses Schritts beträgt ca. 3 bis 30 Minuten, bevorzugt 3 bis 15 Minuten, noch bevorzugter 4 bis 6 Minuten. Die entsprechend gewählten Einstellungen ergeben am Ende des Zentrifugationsschritts ein weißliches Sediment im Röhrchen, über dem ein klarer, gelblicher Überstand zu finden ist.

Anschließend werden die Überstände der zentrifugierten Suspensionen vorsichtig mit einer Pipette abgenommen und in neue Gefäße, z.B. Zentrifugenröhrchen überführt. Es können Überstände mehrerer zentrifugierter Suspensionen zusammengegeben werden. Das Sediment wird verworfen.

Die chromatographische Auftrennung in Schritt (d) erfolgt bevorzugt mittels HPLC-Säulenchromatographie. Hierzu wird die Säule zunächst äquilibriert. Flüssigkeiten zum Äquilibrieren der Säule sind beispielsweise wässrige Lösungen verschiedener organischer oder anorganischer Säuren, bevorzugt ist Trifluoressigsäure. Aber auch andere Lösungsmittel als Wasser sind für die Säuren denkbar, und bevorzugt ist das Lösungsmittel eine wässrige Acetonitril-Lösung. Die Konzentration der jeweiligen Lösungen wird passend eingestellt.

So ist beispielsweise eine Konzentration von 0,01 bis 0,25 Vol.-%, insbesondere 0,05 bis 0,2, besonders bevorzugt 0,1 Vol.-% Trifluoressigsäure in einer 10 bis 30, bevorzugt 15 bis 25, noch bevorzugter eine 20 Vol.-% wässrigen Acetonitril-Lösung gewünscht. Am meisten bevorzugt ist eine Lösung aus 0,1 Vol.-% Trifluoressigsäure in einer 20 Vol.-% wässrigen Acetonitril-Lösung (später als Lösung B bezeichnet). Das Wasser entspricht dem in Schritt (a).

Die Äquilibrierungsflüssigkeit wird mit einer Spritze aufgesogen und diese auf eine Chromatographiesäule gesteckt. Die Lösung (es reichen 15 ml, aber geringe Abweichungen von dieser Menge entsprechend der verwendeten Flüssigkeit sind möglich) wird durch die Säule durchgedrückt. In Rahmen der intensiven Forschungen, die erfindungsgemäßen Fraktionen bereitzustellen, stellte sich heraus, dass eine Sep-Pak-Säule (Waters) mit einer C18-Packung von großer Wichtigkeit war. Es können äquivalente Säulen anderer Hersteller verwendet werden, aber die Packung muss im Wesentlichen einer C18-Säule entsprechen, da überraschenderweise sonst keine oder nur eine geringe Ausbeute verwertbarer Peptide gewonnen werden konnte. Der durch die Säule geflossene Durchfluss, eine klare Lösung, wird verworfen.

Anschließend folgt ein weiterer Äquilibrierungsschritt, der im Wesentlichen dem vorhergehenden entspricht und mit einer Flüssigkeit durchgeführt wird, die im Wesentlichen der Flüssigkeit für die Suspension des Molkepermeats in Schritt (a) entspricht. Bevorzugt ist es genau diese Lösung.

Anschließend werden die oben gewonnenen Überstände aus Schritt (c) mit einer neuen Spritze aufgesogen und langsam und kontinuierlich durch die Säule gedrückt. Dabei verfärbt sich die Säule im oberen Teil mehr oder weniger stark - entsprechend der verwendeten Menge des anfänglich verwendeten Molkepermeats. Auf diese Weise werden die Bestandteile des in Schritt (c) gewonnenen Überstands chromatographisch aufgetrennt. Der Durchfluss wird wieder verworfen.

Als nächstes werden die aufgetrennten Fraktionen eluiert (Schritt (e)). Zuerst wird die hydrophile Peptidfraktion gewonnen. Hierzu wird ein Spritze mit einer im Wesentlichen ähnlichen Lösung befüllt, wie im zweiten Säulen-Äguilibrierungsschritt. Besonders bevorzugt ist die Lösung zum Eluieren die oben genannte Lösung B (0,1% Trifluoressigsäure in 20% wässriger Acetonitril-Lösung). Es ist ausreichend, wenn 1 ml Lösung zum Eluieren verwendet wird, jedoch können auch größere Menge benutzt werden, z.B. 1,5, 2,0 oder 2,5 ml usw.

Die eluierte Fraktion wird in einem Gefäß aufgefangen, z.B. in einem Eppendorf-Röhrchen. Charakteristisch bei einem Volumen von 1 ml Elutionslösung ist, dass zuerst ca. 0,5 ml einer klaren und anschließend 0,5 eines gelblichen Eluats gewonnen werden.

Anschließend wird - ähnlich dem für die hydrophile Fraktion beschriebenen Eluatgewinnung - eine Eluierung der hydrophoben Bestandteile durchgeführt, die in der Säule chromatographisch aufgetrennt wurden. Hierzu wird eine Lösung einer organischen oder anorganischen Säure (z.B. Trifluoressigsäure, 0,1%) in einer höherprozentigen Acetonitril-Lösung (z.B. mindestens 50, besser 60, noch besser 70, bevorzugt einer 80%igen Acetonitril-Lösung in Wasser (nachfolgend Lösung C) in eine Spritze gefüllt und der Inhalt langsam und kontinuierlich durch die Säule gedrückt. Das gelbliche Eluat wird wieder eingesammelt.

Die Äquilibrierungs- und Eluierungsschritte werden so häufig wie gewünscht wiederholt, wobei die gleiche oder eine andere Säule benutzt werden kann. Die gewonnenen Eluate werden zusammengegeben und bilden entweder hydrophile oder hydrophobe Pools. Die Gefäße mit den eluierten Fraktionen werden im geöffneten Zustand in eine Lyophilisierungsvorrichtung gestellt und bei 4°C ins Trockene einrotiert. Zum Lyophilisieren können dem Fachmann bekannte Vorrichtungen verwendet werden.

Der getrocknete Peptidextrakt kann bei -20°C gelagert werden, oder er kann wieder gelöst werden. Hierzu können dem Fachmann bekannte wässrige Puffer oder andere Lösungsmittel verwendet werden. Bevorzugt sind Puffer mit einer Zusammensetzung aus Guanidinium-Hydrochlorid (z.B. 1,0 - 1,5 M, bevorzugt 1,1 M) Tris (50 - 70 mM, bevorzugt 55 mM), NaCl (10 bis 20 mM, bevorzugt 15 mM), KCl (0,70 bis 0,95 mM, bevorzugt 0,88 mM, MgCl₂ (350 bis 450 mM, bevorzugt 400 mM), EDTA (0,5 bis 1,5 mM, bevorzugt 1 mM), GSH (reduziertes Glutathion; 2,0 bis 2,8 mM, bevorzugt 2,4 mM), CaCl₂ (350 bis 450 mM, bevorzugt 400 mM), Polyethylenglykol (5 - 15, bevorzugt 10 mM, pH 8,2 EPuffer -70 mV)), am meisten bevorzugt ist folgende Zusammensetzung: 1,1 M Guanidinium-Hydrochlorid, 55 mM Tris, 15 mM NaCl, 0,88 mM KCl, 400 mM MgCl₂, 1 mM EDTA, 2,4 mM GSH (reduziertes Glutathion), 400 mM CaCl₂, 10 mM Polyethylenglykol (pH 8,2 _{EPuffer} -70 mV)). Ein weiterer bevorzugter Puffer umfasst 10 bis 100 mM, bevorzugt 50 mM Tris/HCl (pH 7,0) in bidestilliertem Wasser.

Die resuspendierten Peptidextrakte können für die funktionalen Experimente verwendet werden.

Die in den oben dargestellten Verfahrenschritten aufgezeigten Alternativen bei den Pufferzusammensetzungen, der Dauer der einzelnen Schritte und der jeweiligen physikalischen Bedingungen (Temperatur, Umdrehungszahlen, Volumina der verwendeten Substanzen usw.) können unterschiedlich miteinander kombiniert werden. Am Ende der jeweiligen Schritte werden Suspensionen oder Flüssigkeiten gewonnen, deren Erscheinungsbild dem Fachmann eine Kontrolle darüber gibt, ob die gewählten Parameter für eine optimale Ausbeute richtig gewählt wurden. Wann immer die erhaltenen Lösungen nicht das beschriebene Aussehen haben, z.B. nicht die gewünschte Farbe (z.B. nach dem Eluieren) oder sie nicht klar sind (z.B. nach dem Resuspendieren des Molkepermeats), ist davon auszugehen, dass suboptimale Bedingungen gewählt wurden, die zwar in geeignet sein können, zu den gewünschten Peptidfraktionen zu führen, jedoch nicht quantitativ und qualitativ dem erfindungsgemäßen Verfahren entsprechen.

Es ist auch möglich einzelne oder mehrere Schritte zu wiederholen, falls dies gewünscht ist.

Eine massenspektrometrische Analyse erfolgt nach dem Fachmann bekannten Verfahren.

Die in den Figuren 1 und 2 gezeigten Peptidfraktionen, die mit dem erfindungsgemäßen Verfahren hergestellt wurden, sind ebenfalls Teil der vorliegenden Erfindung. Ihre Verwendung in den erfindungsgemäßen Zusammensetzungen ist ebenso Bestandteil der Erfindung.

Besonders bevorzugt umfassen die erfindungsgemäßen Peptidfraktionen im Wesentlichen mindestens ein oder mehrere Peptide mit den folgenden Massen:
Massen der Peptide der hydrophoben Fraktion in Dalton: 791, 905, 923, 1023, 1118, 1247, 1391, 1489, 1590, 1702, 1782, 1882, 1980, 2043, 2108, 2206, 2461, 2622, 2785
Massen der Peptide der hydrophilen Fraktion in Dalton: 654, 747, 875, 954, 1041, 1179, 1253, 1338, 1366, 1434, 1500, 1536, 1666, 1698, 1827, 1861, 1986, 2023, 2185, 2315.

Geringfügige Schwankungen der Peptidausbeuten und der Zusammensetzung hängen von der Art des eingesetzten Naturprodukts Süßmolke ab, bilden aber ebenfalls einen Teil der erfindungsgemäßen Fraktionen.

Erfindungsgemäß werden weiter sowohl pharmazeutische Zusammensetzungen oder Medikamente als auch Nahrungsergänzungsmittel bereitgestellt, welche eine oder mehrere der erfindungsgemäßen Fraktionen aus Molkepermeat, insbesondere Süßmolkepermeat umfassen.

Erfindungsgemäße pharmazeutische Zusammensetzungen werden zur Prophylaxe, Behandlung oder Prävention der Glucoseintoleranz und der Insulinresistenz verwendet. Ferner können auf diesem Wege die Symptome des Metabolischen Syndroms oder des Diabetes Typ 2 sowie die damit verbundenen Folgeerkrankungen bei Säugern, bevorzugt beim Menschen, behandelt, gelindert oder verhindert werden.

Beispiele für Folgeerkrankungen, die mit dem Metabolischem Syndrom oder dem Diabetes einhergehen oder deren Entstehen durch diese Erkrankungen begünstigt wird, sind Gefäßerkrankungen, Koronarinsuffizienz, arterielle Verschlusskrankheiten, Mykokardinfarkt, Xanthomen, Bauchbeschwerden, Milz-Leber-Vergrößerung, Pankreatitis, Netzhaut-Lipämie, und/oder Schlaganfall.

Molke fällt bei der Käseherstellung aus Milch an. Erfindungsgemäß kann Kuhmilch, Ziegenmilch, Schafsmilch, Büffelmilch und Kamelmilch zur Molkegewinnung verwendet werden, nachdem das in der Milch vorhandene Kasein ausgefällt wurde. Nach Art der Gewinnung unterscheidet man Süßmolke, die als Milchserum nach enzymatischer Ausfällung von Kasein durch Labferment entsteht, und Sauermolke, welche nach Abtrennung des Kaseins durch Säurefällung gewonnen wird. Die Grenze zwischen den pH-Werten von Süß- und Sauermolke ist nicht ganz scharf umrissen und liegt allgemein über bzw. unter einem Bereich von 5,6 bis 5,9.

Die zur Gewinnung der erfindungsgemäßen Fraktionen verwendete Molke enthält alle wasserlöslichen Komponenten der Milch, sofern diese nicht durch Lab oder Säure ausgefällt werden. In einer besonderen Ausführungsform wird Süßmolke verwendet, die ca. 4,9% Lactose, 0,8% Protein, 0,5% Mineralstoffe, 0,2% Milchfett und 0,2% Milchsäure enthält.

Von der Molke kann mittels Ultrafiltration (Membranverfahren, mittlere Porengröße: 25 bis 100 Kilodalton, kD) das Molkeeiweiß abgetrennt werden. Diese "entweißte" Molke besteht zu etwa 95% aus Wasser und kann durch Sprühtrocknen zu einem Pulver weiterverarbeitet werden kann. Dieses Pulver wird hier als Molkepermeat bezeichnet. In der vorliegenden Erfindung ist die Verwendung von Molkepermeat bevorzugt, das aus Süßmolke gewonnen wurde. Die darin durchschnittlich enthaltenen Bestandteile sind 84.9% Lactose, 4.5% Protein, 0.1% Fett und 7.5% Mineralstoffe. Beim Rest handelt es sich um nicht-abgetrenntes Wasser. Bei diesen Mengenangaben handelt es sich um Durchschnittswerte, die je nach Herstellungsweise um 5-10% (relativ) variieren können.

Molkepermeat kann als solches, oder nach (Teil-)Hydrolyse der Lactose verwendet werden. Bevorzugte Darreichungsformen der Molke sind Sirups oder Pulver, aber auch andere Darreichungsformen sind möglich. Erfindungsgemäß wird die Süßmolke vor der Herstellung der erfindungsgemäßen Fraktionen sowohl hydolysiert als auch teilhydrolysiert verwendet.

In einer weiteren erfindungsgemäßen Ausführungsform hat sich eine Mikroverkapselung der pharmazeutischen Zusammensetzung, die Fraktionen aus Molkepermeat umfasst als besonders vorteilhaft erwiesen. Die Mikroverkapselung kann wie unter anderem in den Patent-Offenlegungsschriften DE 198 54 749 A1 und DE 100 08 880 A1 und dem Gebrauchsmuster DE 296 23 285 U1 beschrieben, erfolgen. Dabei wird die Verbindung zum Beispiel in einer Hülle aus einem Polysaccharid, wie z.B. Alginat, fest eingeschlossen. Damit der möglicherweise unverdauliche Hüllstoff eine Freisetzung der Verbindung nicht verhindert und dadurch eine ernährungsphysiologische Nutzung durch den Organismus unmöglich macht, kann eine verdauliche Komponente, wie z.B. Stärke der Umhüllung beigefügt werden. Durch geschickte Wahl und/oder Kombination der löslichen und unlöslichen Umhüllungskomponenten kann so die Abgabe der mikroverkapselten pharmazeutischen Zusammensetzung in verschiedenen Bereichen des Verdauungstrakts gezielt gesteuert werden. Eine abgestufte Freisetzung im Darm, z.B. eine Freisetzung von 50 bis 80 Gew.-%, bevorzugt von 60 bis 70 Gew.-%, insbesondere von 62,5 Gew.-% im Dünndarm, und eine Freisetzung von 20 bis 50 Gew.-%, bevorzugt von 30 bis 40 Gew.-%, insbesondere ungefähr 35 Gew.-% im Dickdarm ist ein mögliche Art der gezielten Freisetzung. Ein weiterer vorteilhafter Effekt kann durch eine verlängerte Haltbarkeit durch Schutz der verkapselten Verbindung z.B. vor Umwelteinflüssen erzielt werden.

Des Weiteren wird in den Verfahren zur Herstellung der Produkte und Zusammensetzungen die Verwendung von Süßmolkepermeat, bevorzugt aus dem die Lactose teilweise oder ganz entfernt wurde. Dieses Süßmolkepermeat wird hier als Lactose-reduziertes Süßmolkepermeat bezeichnet. Süßmolkepermeat enthält normalerweise ca. 85 % an Lactose, die jedoch daraus zur weiteren Verwendung gewonnen werden kann. Übrig bleibt daher Lactose-reduziertes Süßmolkepermeat, das zwischen 0.1 und 80 %, bevorzugt zwischen 1 bis 50 %, noch bevorzugter zwischen 5 und 25 % an Lactose umfasst. Besonders bevorzugt ist Lactose-reduziertes Süßmolkepermeat, das noch 10 bis 20 % an Lactose umfasst.

Die vorliegende Erfindung stellt ferner Präparate zur Verfügung, welche die folgenden Wirkungen auf Symptome des Metabolischen Syndroms oder des Typ 2 Diabetes haben:
- Verhinderung des Entstehens einer Glucoseintoleranz,
- Verhinderung einer Insulinresistenz,
- Senkung des Seruminsulinspiegels,
- Senkung des Inselvolumens der Bauchspeicheldrüse,
- Verringerung des Volumens der Beta-Zellen im Pankreas,
- Verhinderung der Neogenese von Beta-Zellen im Pankreasgangepithel,
- Verhinderung der Entwicklung von Hyperinsulinismus,
- Verhinderung einer Pankreasentzündung (Pankreatitis) oder einer Insel- und/oder Beta-Zell-selektiven Entzündung (Insulitis).

Ein weiteres Ziel ist die Verwendung der erfindungsgemäßen Zusammensetzungen und Produkte aus Molkepermeat, zur Prävention und/oder Behandlung des Diabetes Typ 2, vor allem zur Verzögerung der Progression der Erkrankung, bevorzugt zur Verbesserung von deren Symptomatik.

Es wurde in WO2005/004990 überraschend entdeckt, dass es für den Hyperinsulinismus ein morphologisches Korrelat gibt. Das bedeutet, dass es zu einer Vermehrung des β-Zellvolumens, dem Auftreten kleiner β-Zellhaufen in der exokrinen Pankreas, dem Nachweis von β-Zellen im Pankreasgangepithel (Hinweis auf eine Neogenese) und eine Erhöhung der Mitoserate der β-Zellen kommt. Dieses morphologische Korrelat spricht für erhöhte Anforderungen an dieses Organ, die partiell durch eine Zellvermehrung kompensiert werden, um beispielsweise die übermäßige metabolisierbare Energie zu verwerten. Es wurde außerdem nachgewiesen, dass die Verabreichung von Molkepermeat zu einer Verhinderung des Anstiegs des β-Zellvolumens führt, und dass diese Substanzen zu einer Verringerung des Inselvolumens in der Pankreas führen. Die Verabreichung der pharmazeutischen Zusammensetzungen und der Nahrungsergänzungsmittel dieser Erfindung für diese Zwecke ist daher bevorzugt in den Schutzbereich eingeschlossen.

Erfindungsgemäß bevorzugt führt die Verwendung von Molkepermeat-Fraktionen zu einer Senkung der Blutfettwerte, insbesondere zu einer Senkung der Triglycerid-Konzentration. Des Weiteren werden pharmazeutische Zusammensetzungen vom Schutzbereich des Patents umfasst, die eine oder mehrere Molkepermeat-Fraktionen und eine oder mehrere weitere wirksame Substanz (en) zur Senkung der Seruminsulinspiegel umfassen. Diese wirksamen Substanzen sind Alpha-Glukosidasehemmer, Biguanide, Glitazone, Sulfonylharnstoffe, Glinide oder Insulin.

Die Verwendung von Molkepermeat-Fraktionen sowie die Verwendung von pharmazeutischen Zusammensetzungen, die neben dem Molkepermeat noch weitere Wirkstoffe enthalten, wird ebenfalls im Rahmen dieser Erfindung zur Verfügung gestellt, solange die weiteren wirksamen Substanzen keinen negativen Einfluss auf die Wirkung der erfindungsgemäßen Fraktionen aus Molkepermeat ausüben.

In einer bevorzugten Ausführungsform werden die Fraktionen aus Molkepermeat zur Prävention oder Behandlung der Glucoseintoleranz und Insulinresistenz sowie des Metabolischen Syndroms oder des Diabetes Typ 2 aus der Milch von Rindern, Ziegen, Schafen, Büffeln, Kamelen oder anderen Milch gebenden Tieren gewonnen.

Eine besonders bevorzugte Ausführungsform ist darauf ausgerichtet, dass Nahrungsmittel Fraktionen aus Molkepermeat enthalten, und in Form von Nahrungsergänzungsmitteln oder von so genanntem "Functional Food" vorliegen. Auch hier ist die Verwendung von Fraktionen aus (teil-)hydrolysiertem Süßmolkepermeat bevorzugt. Nahrungsergänzungsmittel dienen nicht alleine der Sättigung oder dem Genuss, sondern auch dem Erzielen einer gesundheitsfördernden Wirkung, d.h. dem Konsumenten werden mit dem Nahrungsmittel Stoffe zugeführt, die ganz allgemein dazu führen, dass der Organismus gegen negative Einflüsse, z.B. aufgrund von Mängeln an notwendigen Substanzen (z.B. Vitamine, Mineralstoffe etc.) geschützt wird. Die erfindungsgemäßen Nahrungsergänzungsmittel werden zur Prophylaxe und Behandlung von Diabetes und damit assoziierten Folgeerkrankungen bereitgestellt. Die nach dem erfindungsgemäßen Verfahren hergestellten Molkefraktionen sind in diesem Zusammenhand besonders bevorzugt.

In einer weiteren bevorzugten Ausführungsform werden die Fraktionen aus Molkepermeat zur Herstellung von diätetischen und/oder angereicherten Nahrungsmitteln, bzw. Nahrungsergänzungsmitteln verwendet, die geeignete pharmazeutisch annehmbare Zusatzstoffe umfassen. Beispiele für Zusatzstoffe sind dem Fachmann bekannte Farbstoffe, Geschmacksverstärker, Konservierungsmittel, Bindemittel, oder Füllstoffe.

Erfindungsgemäß können die oben genannten Fraktionen aus Molkepermeat auch zur Herstellung von Nahrungsmitteln, zum Beispiel diätetischen Nahrungsmitteln und/oder Nahrungsergänzungsmitteln verwendet werden, wodurch diese die Eigenschaft erhalten, die Glucoseintoleranz und/oder Insulinresistenz, und gegebenenfalls dadurch das Metabolische Syndrom oder die Progression des Typ 2-Diabetes zu verhindern und/oder die Symptome der letztgenannten Indikationen zu lindern oder zu heilen. Beispiele für solche Nahrungs(ergänzungs)mittel sind Milchprodukte oder Fruchtsäfte, die mit erfindungsgemäßen Zusammensetzungen angereichert sind, aber auch andere Nahrungsmittel sind denkbar.

Zur Behandlung und Prävention von Insulinresistenz und/oder Glucoseintoleranz, insbesondere beim Metabolischen Syndrom und Typ 2-Diabetes, mit Hilfe von Zusammensetzungen oder Präparaten, die Fraktionen aus Molkepermeat enthalten, wird die Dosierung je nach Krankheitsindikation, Alter, Gewicht und Geschlecht der zu behandelnden Person gegebenenfalls individuell variiert.

Die Konzentration der erfindungsgemäßen Zusammensetzungen oder Präparate in diätetischen und/oder angereicherten Nahrungsmitteln, die Fraktionen aus Molkepermeat enthalten, kann in Abhängigkeit vom gewählten geeigneten Zusatzstoff und/oder Trägerstoff variieren, wobei die organoleptischen Eigenschaften des Endprodukts besonders berücksichtigt werden.

Des Weiteren können die erfindungsgemäßen Zusammensetzungen in einem Kombinationspräparat zur Prävention oder Behandlung von Symptomen des Metabolischen Syndroms und des Typ 2-Diabetes enthalten sein. Besonders bevorzugt enthält ein Kombinationspräparat neben den erfindungsgemäßen Molke-Fraktionen eine oder mehrere der folgenden Substanzen: Alpha-Glukosidasehemmer, Biguanide, Glitazone, Glinide, Sulfonylharnstoffe. Die Verabreichung der Kombinationspräparate kann ebenfalls individuell an die Bedürfnisse des Patienten angepasst werden. Erfindungsgemäß können diese Kombinationspräparate einzeln in 2 oder mehr Einzeldosierungen oder zusammen verabreicht werden.

Besonders bevorzugt ist außerdem die Verwendung einer erfindungsgemäßen Zusammensetzung zur Herstellung eines Medikaments, bevorzugt zur Linderung oder Verhinderung der Glucoseintoleranz und/oder Insulinresistenz, insbesondere als Symptome des Metabolischen Syndroms und der Behandlung und Verhinderung des Entstehens von Typ 2-Diabetes, der Verhinderung von deren Progression sowie von Folgeerkrankungen, die durch das Metabolische Syndrom oder den Diabetes entstehen können.

Die Verwendung der erfindungsgemäßen Zusammensetzungen oder Medikamente zur Linderung/Prävention von Glucoseintoleranz und/oder Insulinresistenz, welche insbesondere Symptome des Metabolischen Syndroms und des Typ 2-Diabetes sind, ist besondere auf die Anwendung am Menschen ausgerichtet, aber die Behandlung anderer Säugetiere wie Hunde, Katzen, Pferde, Schafe, Kühe oder Schweine etc. ist ebenfalls in den Schutzbereich eingeschlossen.

Die erfindungsgemäßen Zusammensetzungen und Präparate werden besonders bevorzugt zur Bekämpfung/Prävention des Wachstums von β-Zellen im Pankreas, der Vermehrung von β-Zellen im Pankreas, dem Anstieg des β-Zell- bzw. Inselvolumens oder der Pankreatitis bei einem Säugetier, insbesondere dem Menschen, verwendet.

Des Weiteren ist es besonders bevorzugt, dass die Verwendung der erfindungsgemäßen Zusammensetzungen oder Präparate zu einer Senkung des Seruminsulinspiegels führt.

Insbesondere bevorzugt wird, dass durch die Gabe der erfindungsgemäßen Zusammensetzungen oder Präparate das Entstehen einer Glucoseintoleranz verhindert wird, oder dass diese gelindert wird.

Bevorzugt wird außerdem, dass die Verabreichung der erfindungsgemäßen Zusammensetzungen oder Präparate zu einem verzögerten Anstieg der morgendlichen Hyperglykämie führt.

Weiterhin wird besonders bevorzugt, dass das Konsumieren der erfindungsgemäßen Zusammensetzungen oder Präparate zu einer Senkung der Blutfettwerte führt, insbesondere der Senkung der Konzentration an Triglyceriden, jedoch ist eine Beeinflussung anderer Blutfette ebenfalls in den Schutzbereich der vorliegenden Erfindung einbezogen.

Erfindungsgemäß wird den pharmazeutischen Zusammensetzungen oder Präparaten, die Fraktionen aus Molkepermeat umfassen, kein Calciumlactat hinzugegeben.

Von einer Hypertriglyceridämie (Hyperlipämie) spricht man bei einem erhöhten (> 160 mg/100ml) Triglyceridgehalt im Blutserum. Dieser Zustand kann eine Rolle bei der Entstehung von Arterienerkrankungen und der Entwicklung einer koronaren Herzkrankheit spielen (Vega und Grundy, Adv. Exp. Med. 243, 311 (1989)). Zusätzlich ist eine schwere Hypertriglyceridämie (>1.000 mg/dl) mit Chylomicronämie verbunden und sie ruft akute Pankreatitis hervor (siehe K. Soergel, Acute Pancreatitis, in Gastronintestinal Disease 91, 3. Ausgabe (Sleisenger, M.H. und Fordtran, J.S., Hrsg.), W.B. Saunders Company, Philadelphia, Pa., 1983, S. 1462-1485; und Brown, M.S. und Goldstein, J.L., Drugs used in the Treatment of Hyperlipoproteinemias, in Goodman and Gillman's, The Pharmacological Basis of Therapeutics 34, 7. Ausgabe, (Macmillan Publishing Co., New York, 1985, S. 827-845).

Ernsthaft erhöhte Konzentrationen der Chylomicrone rufen auf direktem Weg eine Pankreatitis hervor, die durch Triglyceridverringerung verhindert werden könnte (U.S. Department of Health and Human Services, NIH-Publication Nr. 89-2925, S. 74-77, Jänner 1989, "Report of the Expert Panel on Detection, Evaluation and Treatment of High Blood Cholesterol in Adults"). Es hat sich außerdem gezeigt, dass das Risiko eines Schlaganfalls bei Patienten mit koronaren Herzkrankheiten durch Verringerung des Triglyceridgehalts im Plasma vermindert werden kann (D. Tanne, et al.; Circulation 2001, 104, 2892-2897). Weiterhin ist bei Typ 2-Diabetikern die Lebenserwartung gegenüber Nichtdiabetikern um ein Drittel verkürzt. Dies steht vermutlich in direktem Zusammenhang mit einem erhöhten Triglyceridspiegel (Diabety Care; 2001- 24, 1335-1341). Es ist daher wünschenswert im Rahmen der Behandlung von Symptomen der Glucoseintoleranz und Insulinresistenz sowie des Metabolischen Syndroms und des Typ 2-Diabetes auch ein Verfahren zur Verringerung von Plasmatriglyceriden bei Patienten mit Hypertriglyceridämie bereitzustellen.

Besonders bevorzugt ist die Verabreichung der erfindungsgemäßen Zusammensetzungen oder Präparate auf oralem Wege, aber auch andere Darreichungsmöglichkeiten sind in den erfindungsgemäßen Schutzbereich einbezogen, z.B. parenteral, intravenös, etc.

Die Konzentration an Fraktionen aus Molkepermeat in einem pharmazeutischen oder Nahrungsergänzungsmittelpräparat liegen zwischen 0,01% und 99,99%, bevorzugt zwischen 0,1 und 99,9%, bevorzugter zwischen 1 und 99%, insbesondere bevorzugt zwischen 1 und 80%, noch bevorzugter zwischen 10 und 80%, am meisten bevorzugt zwischen 10 und 50% bezogen auf das Gesamtgewicht des Nahrungsergänzungsmittels und/oder des pharmazeutischen Präparats.

Die erfindungsgemäßen Nahrungsergänzungsmittel, bzw. die erfindungsgemäßen pharmazeutischen Präparate können ein oder mehrmals täglich eingenommen bzw. verabreicht werden, z.B. morgens, mittags, abends, zusammen, vor oder nach den Mahlzeiten, aber auch andere Darreichungsschemata sind vorstellbar.

In einer besonders bevorzugten Ausführungsform der vorliegenden Erfindung, werden die pharmazeutischen Zusammensetzungen oder Nahrungs(ergänzungs)mittel verabreicht, um die Entstehung reaktiver Sauerstoffspezies zu verhindern, bzw. deren negative Folgen für den Organismus zu schwächen oder zu eliminieren. Unter reaktiven Sauerstoffspezies werden erfindungsgemäß Superoxid, Wasserstoffperoxid und Hydroxylradikal verstanden.

Die Erfindung wird im Folgenden durch Experimente näher erläutert, welche nicht den Bereich der Erfindung weiter einschränken sollen.

### Experimenteller Teil

### I. Aufreinigung von Molkepeptidfraktionen aus Süßmolkepermeat

Süßmolkepermeat wurde durch Ultrafiltration über ein Hohlfaser-Tangentialstromfilter mit einem cut-off von 10 kDa hergestellt. Dadurch ist der Proteingehalt reduziert, weil nur Proteine/Peptide, die kleiner als 10 kDa sind, den Filter passieren können.

Zur Aufreinigung der Peptidfraktionen wurden:
(a) 5 g gepulvertes Süßmolkepermeat in 125 ml der Lösung A (0,1% Trifluoressigsäure in Merck-Wasser entsprechend 500 µl Trifluoressigsäure, Fluka Kat.Nr. 91699 in 500 ml Wasser, VMR Kat. Nr. 1.15333.2500) suspendiert. Hierzu werden die Reagenzien ca. 30 min bei 400 Upm bei 25°C in einem 250 ml Becherglas (Schott Duran) auf einem Magnetrührer gemischt bis kein Bodensatz mehr zu erkennen ist.
(b) Danach wird das das Becherglas mit der darin enthaltenen Suspension freischwimmend im Ultraschallbad (Sonorex RK 156, Bandelin (35kHz, 120W) für 3 min mit maximaler Leistung (60W) behandelt. Die Wasserbadtemperatur beträgt 15°C.
(c) Anschließend wird die Suspension in 12,5 ml Röhrchen (Falcon) pipettiert um mit 14,000g für 5 min bei 25°C zentrifugiert (Ultra-Zentrifuge Discovery Hitachi Sorvall 90 SE, Rotor T 890). Beim Zentrifugieren bildet sich ein weißliches Sediment und ein gelblicher, klarer Überstand.
(d) Die Überstände der zentrifugierten Suspensionen werden mit einer Pipette abgenommen und zusammengegeben. Das Sediment wird verworfen.
(e) Dann wird eine neue 20 ml Spritze (Terumo Syringe 20 ml, SS+T20ES) mit 15 ml Lösung B (0,1% Trifluoressigsäure in 20% Acetonitril entsprechend 0,5 Trifluoressigsäure + 100 ml Acetonitril, VWR Kat.-Nr. 1.00 030.2500 ad 500 ml bidestilliertes Wasser) befüllt. Die Spritze wird auf eine Sep Pak-Säule (C-18, Waters, Part. No.: WAT051910) gesteckt und die enthaltene Lösung B wird durch die Sep Pak-Säule gedrückt. Bei jeder Durchführung dieses Schritts wird die Spritze auf das kurze Ende der Sep Pak-Säule aufgesteckt. Der Durchfluss, eine klare Lösung, wird verworfen.
(f) Danach wird eine neue 20 ml Spritze mit 15 ml Lösung A aufgezogen, auf das kurze Ende der Sep Pak-Säule gesteckt und durch die Säule gedrückt. Der Durchfluss wird verworfen.
(g) Jetzt werden 15 ml des oben in (d) gewonnenen Zentrifugationsüberstands mit einer neue 20 ml Spritze aufgezogen und langsam und kontinuierlich durch die Sep Pak-Säule gedrückt. Die Säule verfärbt sich dabei im oberen Teil gelblich. Der Durchfluss wird wiederum verworfen.
(h) Anschließend wird die hydrophile Peptidfraktion im Durchfluss eluiert. Hierzu wird eine 1 ml Spritze (Omnifix F 1ml, Roth Kat.-Nr. H999) mit 1 ml Lösung B befüllt und durch die Sep Pak-Säule gedrückt. Der Durchfluss wird in einem 2 ml Reaktionsgefäß (Eppendorf Safe-Lock, 2 ml, Kat.-Nr. 0030120.094) aufgefangen. Zu Beginn dieses Schritts eluieren ca. 0,5 ml einer farblosen Flüssigkeit, anschließend treten ca. 0,5. ml gelbliches Eluat aus der Sep. Pak-Säule).
(i) Danach wird die hydrophobe Peptidfraktion im Durchfluss eluiert. In eine neue 1 ml Spritze werden 1 ml Lösung C (0,1 % Trifluoressigsäure in 80% Acetonitril, entsprechend 0,5 ml Trifluoressigsäure + 400 ml Acetonitril ad 500 ml bidestilliertes Wasser) gefüllt und langsam und kontinuierlich durch die Sep Pak-Säule gedrückt. Der Durchfluss wird in einem 2 ml Reaktionsgefäß gesammelt. Es eluiert eine gelbliche Flüssigkeit.
(j) Die Schritte (f) bis (i) wiederholt, bis die in Schritt (d) gewonnenen Überstände vollständig verbraucht sind. Hierzu kann die gleiche Sep Pak-Säule verwendet werden.
(k) Jetzt werden alle erhaltenen hydrophilen bzw. hydrophoben Peptidfraktionen zusammen gegeben.
(l) Die Gefäße, die die hydrophilen bzw. hydrophoben Peptidfraktionen enthalten werden in geöffnetem Zustand in eine SpeedVac (Univapo 150 H, UniEquib 5) gestellt und bei 4°C mit zur Trockene einrotiert.
(m) Der getrocknete Peptidextrakt kann nun sofort im Nativpuffer 1 (1,1 M Guanidinium-Hydrochlorid, 55 mM Tris, 15 mM NaCl, 0,88 mM KCl, 400 mM MgCl₂, 1 mM EDTA, 2,4 mM GSH (reduziertes Glutathion), 400 mM CaCl₂, 10 mM Polyethylenglykol (pH 8,2 EPuffer -70 mV))) oder Nativpuffer 2 (50 mM Tris/HCl (pH 7.0) in bidestilliertem Wasser) resolubilisiert werden und beispielsweise für funktionale Analysen eingesetzt werden. Das Peptidpellet kann auch in anderen wasserlöslichen Puffern aufgenommen und weiterverarbeitet werden. Gegebenenfalls kann der getrocknete Peptidextrakt bei -20°C gelagert werden.

Die qualitative Ausbeute der hydrophoben Peptidfraktionen aus 5 g Süßmolkepermeat betrug ca. 30 mg (- 0,6%). Die Ausbeute der hydrophilen Peptidfraktion bei der gleichen Ausgangsmenge Süßmolkepermeat, betrug ca. 9 mg (- 0,18%).

### 2. Massenspektroskopie-Analyse (MS-Analyse) der gewonnenen Peptidfraktionen

Die erhaltenen hydrophilen bzw. hydrophoben Peptidfraktionen wurden massenspektrometrisch (Voyager - STR; Reflektor-Mode) qualitativ untersucht. Zur MS-Analyse wurde von jeder Fraktion ca. 1µg der Peptidfraktionen eingesetzt. Die Figuren 1 bzw. 2 zeigen die Ergebnisse der MS-Analysen der hydrophilen bzw. hydrophoben Peptidfraktionen.

### 3. In vitro Untersuchung der gewonnenen Peptidfraktionen an Modellen mit besonderer Relevanz für Vorgänge beim Diabetes

Um die Wirksamkeit der untersuchten Proben zu ermitteln, muss gewährleistet sein, dass sich in den Reaktionsansätzen der biochemischen Testsysteme gleiche Mengen der zu testenden Substanz befinden. Bezugsgröße hierfür ist der Trockenmassegehalt. Für Dosis-Wirkungskurven wurden Trockenmassegehalte von 0,1 - 20 mg eingesetzt.

Beim getesteten SMP handelt es sich um ein ultrafiltriertes und sprühgetrocknetes Molkepermeat aus frischer Süßmolke. Nach Angaben des Herstellers (Hafen-Mühlen-Werke GMBH, Bremen, Deutschland) setzt sich dieses Produkt wie folgt zusammen:
Lactose: min 83,0%
Protein: 2,0 . 5,0%
Mineralien : max. 9,0%
Fett: max. 1,5%
Feuchtigkeit: max. 3,0%

### 3. Verwendete Diabetes-Modellsysteme:

### 3.1 DHF-System

Bei erhöhter Insulinresistenz (Typ 2-Diabetes) in der Leber ist die Glukoneogenese und Glukoseabgabe in das Blut trotz Hyperglykämie nicht verringert, sondern gesteigert. Die Glukose durchläuft bevorzugt inaktive, so genannte frustrane Stoffwechselzyklen, ohne oxidiert (Abbau) oder polymerisiert (Bildung von Glycogen) zu werden. Dies eröffnet die Möglichkeit einer Zuckerautooxidation in Gegenwart von Spuren an Übergangsmetallen, welche mit der Bildung reaktiver Sauerstoffspezies (Superoxid, Wasserstoffperoxid, Hydroxylradikal) einhergeht.

Die Autooxidation von Monosachariden ist eine sehr langsame Reaktion. Der geschwindigkeitsbestimmende Schritt ist die Bildung von Endiol. Monosacharide wie Glukose reduzieren über ihr Endioltautomer Sauerstoff zu Superoxid. Dihydroxyfumarsäure (DHF) ist ein Endiol und kann als Modellverbindung für autooxidierende Zucker dienen.

DHF wird im Modellsystem in Anwesenheit von Cu-Ionen oxidiert. Dabei entstehen als reaktive Sauerstoffspezies vorrangig Superoxid, aber auch Wasserstoffperoxid und das aggressive Hydroxylradikal. Die reaktiven Sauerstoffspezies reagieren mit dem Indikatormolekül KMB (α-Keto-S-Methyl-Buttersäure), das u.a. zu Ethen zerfällt, welches gaschromatographisch sehr empfindlich detektiert werden kann. Eine verminderte Freisetzung von Ethen aus KMB in Anwesenheit von Testsubstanzen ist mit einer Entgiftung der reaktiven Sauerstoffspezies gleichzusetzen.

Einer Entgiftung des Superoxids kommt eine entscheidende Bedeutung zu, denn zum einen dient Superoxid als Vorläufermolekül für die Entstehung von Wasserstoffperoxid und von Hydroxylradikalen, zum anderen ist es an der Bildung des Peroxynitrits beteiligt. Superoxid reagiert in einer extrem schnellen Reaktion mit NO unter Generierung des Peroxynitrits. Diese Verbindung ist selbst ein potentes Oxidans und entsteht unter Verbrauch von NO, einem endogenen Gefäßrelaxans und einem Inhibitor der Adhäsion von Blutmonozyten am Gefäßendothel. Diese beiden Funktionen von NO wirken der Atherosklerose entgegen.

Folgender Reaktionsansatz wurde benutzt:

| Reagenz | Konzentration im Ansatz |
|---|---|
| Phosphat-Puffer, pH 7,4 | 100 mM |
| Dihydroxyfumarsäure | 0,25 mM |
| Kupfer (II) Sulfat | 0,005 mM |
| Testsubstanzen | 0, 0.1-20 mg |
| KMB | 1, 5 mM |
| H2O (bidest.) ad 2 ml | |

Die Reaktionsansätze werden in volumengeeichten, gasdicht verschlossenen Reaktionsgefäßen 30 Minuten bei 37°C inkubiert. Anschließend erfolgt mittels Headspace-Technik die gaschromatographische Quantifizierung der während der Reaktion freigesetzten Mengen an Ethen.

### 3.2 Peroxynitrit-System

Peroxynitrit ist ein potentes zytotoxisches Oxidans, das aus der Reaktion von Superoxid mit NO hervorgeht. Durch entzündliche Prozesse angeregte Immunzellen (Makrophagen) produzieren sowohl NO als auch Superoxid, so dass Peroxynitrit an allen Entzündungsherden entsteht. Ein weiterer gemeinsamer Entstehungsort der Vorläufermoleküle für Peroxynitrit ist das Blutgefäßsystem, wo das Endothel NO und durch Xanthinoxidase oder autooxidierende Zucker Superoxid freigesetzt wird.

Ein grundlegender Mechanismus der Toxizität von Peroxynitrit liegt in der Einführung von Nitrogruppen (Bildung von Nitrotyrosin) in Proteine, die damit ihre Funktion verlieren.

Nitrotyrosin konnte sowohl in Inselzellen als auch im Blutgefäßsystem von Diabetikern nachgewiesen werden. Im Tiermodell wurde vor kurzem gezeigt dass eine Substanz, die Peroxynitrit zerstören kann, sowohl die Entwicklung eines Diabetes als auch kardiovaskuläre Komplikationen unterdrücken konnte (Delaney et al, FEBS Letters 394 (1996 300-306; Sensitivity of pancreatic islets to peroxynitrite-induced cell dysfunction and death). Das weist auf die zentrale Rolle von Peroxynitrit bei der Auslösung der Erkrankung und der Manifestation der Spätkomplikationen hin. Peroxynitrit kann, wie auch andere starke Oxidantien, KMB spalten wobei Ethen freigesetzt wird. Im Modellsystem wird synthetisch hergestelltes Peroxynitrit verwendet. Eine geringere Freisetzung von Ethen aus KMB in Anwesenheit der Testsubstanzen ist mit einer Entgiftung des Peroxynitrits gleichzusetzen.

### Folgender Reaktionsansatz wurde verwendet:

| Reagenz | Konzentration im Ansatz |
|---|---|
| Phosphat-Puffer, pH 7,4 | 100 mM |
| Synthetisches Peroxynitrit | 25 µM |
| Testsubstanzen | 0, 0,1-20 mg |
| KMB | 1, 5 mM |
| H2O (bidest.) ad 2 ml | |

Die Reaktionsansätze werden wie im ersten System in volumengeeichten, gasdicht verschlossenen Reaktionsgefäßen 30 Minuten bei 37°C inkubiert. Anschließend erfolgt mittels Headspace-Technik die gaschromatographische Quantifizierung der während der Reaktion freigesetzten Mengen an Ethen.

### 3.3 X/XOD-System

Superoxid kann auch enzymatisch generiert werden. Das Enzym Xanthinoxidase (XOD) reduziert Sauerstoff auf Kosten von Xanthin (X) zum Superoxidradikalanion und zu Wasserstoffperoxid. In Anwesenheit von Übergangsmetall-Ionen (z.B. Fe²⁺), die in Spuren in jedem Puffer bzw. der Enzympräparation vorliegen, kann Wasserstoffperoxid zum aggressiven OH-Radikal reduziert werden.

Fe²⁺ selbst geht dabei in Fe³⁺ über, welches durch Superoxid aber wieder zum Fe2+ regeneriert wird. Diese Reaktionssequenz wird als Haber-Weiss-Reaktion bezeichnet, die als eine der bedeutendsten Quellen für reaktive Sauerstoffspezies (ROS) in vivo gilt. Sowohl Superoxidradikalanionen als auch OH-Radikale sind wie schon erwähnt in der Lage, α-Keto-S-Methyl-Buttersäure (KMB) anzugreifen und dieses Molekül oxidativ zu spalten. Dabei entsteht als Spaltprodukt Ethen, das gaschromatographisch sehr empfindlich nachgewiesen werden kann.

In vivo ist die XOD besonders in schlecht durchbluteten Geweben und Gefäßen aktiv, z.B. unter Bedingungen atherosklerotischer Veränderungen, welche eine Hauptkomplikationen des Diabetes mellitus darstellen.

### Folgender Reaktionsansatz wurde benutzt:

| Reagenz | Konzentration im Ansatz |
|---|---|
| Phosphat-Puffer, pH 7,4 | 100 mM |
| XOD | 0,04 U |
| Xanthin | 0,5 mM |
| Testsubstanzen | 0, 0,1-20 mg |
| KMB | 1,5 mM |
| H2O (bidest.) ad 2 ml | |

Die Reaktionsansatze werden in volumengeeichten, gasdicht verschlossenen Reaktionsgefäßen 30 Minuten bei 37°C inkubiert. Danach erfolgt mittels Headspace-Technik die gaschromatographische Quantifizierung der während der Reaktion freigesetzten Mengen an Ethen.

### 3.4 Bestimmungen der IC50-Werte

Für den direkten Vergleich der Wirksamkeit verschiedener Substanzen in einem Modellsystem kann der IC50-Wert herangezogen werden. Der IC50-Wert bezeichnet die Konzentration einer Substanz im Ansatz, die zu einer halbmaximalen Hemmung der Kontrollreaktion (ohne Substanz) führt. Die IC50-Werte werden aus der Graphik mit Hilfe eines Screen-Readers abgelesen. Dazu wird jede Kurve in der Graphik einer sigmoiden Anpassung unterzogen. Auf Höhe der 50%-Marke der Y-Achse wird eine Gerade parallel zur x-Achse gelegt. Der Schnittpunkt dieser Geraden mit der angepassten Kurve ergibt die Konzentration, die dem IC50-Wert entspricht.

### 3.5. Ergebnisse

### 3.5.1 Das DHF-System

Durch die Zugabe von SMP (Hasan) wird eine Hemmung der Bildung von Ethen und damit eine Entgiftung der in diesem System gebildeten reaktiven Verbindungen bis zu 20% beobachtet. Ab einer Konzentration von 1 mg im Ansatz weist SMP hervorragende Aktivität als Antioxidans aus.

Um auf die wirksamen Bestandteile des Molkepermeats in den biochemischen Modellsystemen schließen zu können, wurden von SMP Unterfraktionen hergestellt und im Testsystem untersucht.. Die Konzentrationen dieser Bestandteile wurden so gewählt, dass sie ihrem Anteil an der Trockensubstanz des SMP entsprechen. Hydrophobe Bestandteile (Peptide und Proteine) zeigten hier eine besonders gute Wirksamkeit. In absoluten Zahlen ausgedruckt: 75 ng hydrophobe Peptide sind in der Lage, die Bildung reaktiver Verbindungen im DHF-System um fast 60% zu hemmen.

### 4.2 Das Peroxynitrit-System

Im Peroxynitrit-System erwies sich SMP als nicht so wirkungsvoll wie im DHF-System, wenngleich es bei hohen Konzentrationen im Ansatz (20 mg) Peroxynitrit zu etwa 60% entgiftet. Unter den Bestandteilen des SMP erwiesen sich die hydrophoben Bestandteile als am wirkungsvollsten.

### 4.3 Das X/XOD-System

In diesem System besitzt SMP ausgeprägte antioxidative Eigenschaften. Bei einer Konzentration von 1 mg im Ansatz kann das X/XOD System von SMP schon zu über 50% gehemmt werden. Auch die hydrophoben Bestandteile und die Zuckerfraktion bewirkten eine starke Hemmung.

Für die Wirkung der hydrophoben Peptidfraktion kann es sich aufgrund der extrem niedrigen Konzentration (nur 0,45 µg) nicht um einen unspezifischen Radikalfänger-Effekt handeln.

Die Ergebnisse aus dem DHF-System zeigen viele Übereinstimmungen mit dem X/XOD-System. SMP kann das System bei 10 mg im Ansatz zu ca. 90% hemmen. Die IC50-Werte fallen nicht ganz so niedrig aus, liegen aber mit maximal 2,31mg im Ansatz immer noch auf sehr niedrigem Niveau. Auch die hydrophobe Peptidfraktion erwies sich als sehr wirksam.

Besonders interessant sind die Resultate aus dem Peroxynitrit-System. Peroxynitrit ist kein Radikal. Es reagiert bevorzugt mit Proteinen. SMP kann bei dieser hohen Konzentration immerhin eine 60%ige Hemmung bewirken. Auch die hydrophoben Bestandteile besitzen eine gute Entgiftungsfunktion gegenüber Peroxynitrit.

### 5. Princess NINA-NFκB-Reportergen-Assay

Zusätzlich zu den oben dargestellten Untersuchungen wurden SMP und Fraktionen davon in einem NFκB-Reportergen-Assay (Cell Culture Service, Hamburg, Deutschland) untersucht.

Dieser Assay basiert auf folgenden Grundlagen. Infolge eines entzündlichen Stimulus durch TNF-α wird in der Zelllinie A549-NFkB (CCS, Cell Culture Services, Hamburg, Deutschland) durch Aktivierung des NFkB-Signalwegs die Expression von SEAP (Sektretierte Embryonale Alkalische Phosphatase) induziert. SEAP kann als Maß für die Aktivierung leicht im Überstand der Zellen quantifiziert werden. Hemmen die Testsubstanzen in ihrer Einsatzkonzentration die Aktivierung von NFκB, so ist dieses durch die verminderte Expression von SEAP messbar.

Zur Untersuchung der Wirksamkeit werden verschiedene Konzentrationen der zu testenden Substanzen verwendet. Die maximale Konzentration betrug 10 % der Trockenmasse, welche in 6 Schritten jeweils 1:2 mit Zellkulturmedium der A549-Zellen verdünnt wurde. Die A549-NFkB werden in 96 wellPlatten ausgesät und 20 Stunden mit der Testsubstanz vorinkubiert. Als Positivkontrolle, die eine starke Hemmung der Genexpression bewirkt, wurde 5mM Dexamethason eingesetzt. Anschließend wurden die Zellen mit TNF-α (10ng/ml) stimuliert, um die inflammatorische Reaktion zu simulieren. Die Zellen wurden für weitere 24 Stunden beim 37°C, 5% CO2 und hoher Luftfeuchtigkeit kultiviert.

Als Maß für die Aktivierung des NFκB-Signalwegs wurde die Expression des SEAP-Reportergens im Kulturüberstand bestimmt. CSPD^{®} wird von SEAP als Substrat in ein chemilumineszierendes Produkt umgesetzt, da in eine Plattenluminometer (Genios, Tecan) quantifiziert werden kann. Um Verfälschungen der Ergebnisse durch toxische Einflüsse der Prüfsubstanzen auf die Zellen auszuschließen, wurden diese mit dem Vitalfarbstoff Resazurin inkubiert. Metabolisch aktive Zellen setzen das blaue Resazurin das fluoreszenzaktive Resofurin um. Die Umsetzung kann mittels eines Fluoreszenz-Readers (Genios, Tecan) bei einer Anregung von 560 nm und einer Emissionswellenlänge von 590 nm bestimmt werden. Bei der Auswertung werden die Werte auf den Bereich zwischen den unstimulierten Zellen (0%) und den stimulierten (100%) Kontrollzellen normiert.

In den Untersuchungen wurde festgestellt, dass durch SMP und die erfindungsgemäße hydrophobe Peptidfraktion konzentrationsabhängig eine Hemmung der Aktivierung des NFKB-Signalwegs durch TNF-a möglich ist

## Patentansprüche

1. Isolierte Fraktion aus Süßmolkepermeat, umfassend ein oder mehrere Polypeptide mit einer Masse von 100 bis 3500 Dalton, vorzugsweise 200 bis 3000 Dalton.

2. Isolierte Fraktion aus Süßmolkepermeat gemäß Anspruch 1, umfassend hydrophobe Polypeptide mit einer Masse von 500 bis 3000 Dalton.

3. Isolierte Fraktion aus Süßmolkepermeat gemäß Anspruch 1, umfassend ein oder mehrere hydrophile Polypeptide mit einer Masse von 500 bis 2500 Dalton.

4. Isolierte Fraktion aus Süßmolkepermeat gemäß irgendeinem der Ansprüche 1 bis 3, **dadurch gekennzeichnet, dass** das Süßmolkepermeat hydrolysiert oder teilhydrolysiert ist.

5. Pharmazeutische Zusammensetzung umfassend eine isolierte Fraktion aus Süßmolkepermeat gemäß irgendeinem der Ansprüche 1 bis 4.

6. Isolierte Fraktion aus Süßmolkepermeat gemäß irgendeinem der Ansprüche 1 bis 4 zur Verwendung als Medikament.

7. Isolierte Fraktion aus Süßmolkepermeat gemäß irgendeinem der Ansprüche 1 bis 4 zur Verwendung bei der Prävention oder Behandlung von Typ 2- Diabetes, Metabolischem Syndrom oder Folgeerkrankungen davon.

8. Verwendung mindestens einer isolierten Fraktion aus Süßmolkepermeat gemäß irgendeinem der Ansprüche 1 bis 4 zur Herstellung eines Medikaments zur Prävention oder Behandlung von Typ 2- Diabetes, Metabolischem Syndrom oder Folgeerkrankungen davon.

9. Nahrungsergängzungsmittel, umfassend mindestens eine der isolierten Fraktionen aus Süßmolkepermeat gemäß irgendeinem der Ansprüche 1 bis 4.

## Claims

1. Isolated fraction of sweet whey permeate comprising one or more polypeptides having a mass of 100 to 3,500 Dalton, preferably 200 to 3,000 Dalton.

2. Isolated fraction of sweet whey permeate according to claim 1 comprising hydrophobic polypeptides having a mass of 500 to 3,000 Dalton.

3. Isolated fraction of sweet whey permeate according to claim 1 comprising one or more hydrophilic polypeptides having a mass of 500 to 2,500 Dalton.

4. Isolated fraction of sweet whey permeate according to any of claims 1 to 3, **characterised in that** the sweet whey permeate is hydrolysed or partly hydrolysed.

5. Pharmaceutical composition comprising an isolated fraction of sweet whey permeate according to any of claims 1 to 4.

6. Isolated fraction of sweet whey permeate according to any of claims 1 to 4 for use as a medicament.

7. Isolated fraction of sweet whey permeate according to any of claims 1 to 4 for use in the prevention or treatment of Type 2 diabetes, metabolic syndrome or secondary diseases thereof.

8. Use of at least one isolated fraction of sweet whey permeate according to any of claims 1 to 4 for the production of a medicament for the prevention or treatment of Type 2 diabetes, metabolic syndrome or secondary diseases thereof.

9. Food supplement comprising at least one of the isolated fractions of sweet whey permeate according to any of claims 1 to 4.

## Revendications

1. Fraction isolée de perméat de lactosérum doux, comprenant un ou plusieurs polypeptides présentant une masse de 100 à 3500 daltons, de préférence de 200 à 3000 daltons.

2. Fraction isolée de perméat de lactosérum doux selon la revendication 1, comprenant des polypeptides hydrophobes présentant une masse de 500 à 3000 daltons.

3. Fraction isolée de perméat de lactosérum doux selon la revendication 1, comprenant un ou plusieurs polypeptides hydrophiles présentant une masse de 500 à 2500 daltons.

4. Fraction isolée de perméat de lactosérum doux selon l'une quelconque des revendications 1 à 3, **caractérisée en ce que** le perméat de lactosérum doux est hydrolysé ou est partiellement hydrolysé.

5. Composition pharmaceutique comprenant une fraction isolée de perméat de lactosérum doux selon l'une quelconque des revendications 1 à 4.

6. Fraction isolée de perméat de lactosérum doux selon l'une quelconque des revendications 1 à 4, pour son utilisation comme médicament.

7. Fraction isolée de perméat de lactosérum doux selon l'une quelconque des revendications 1 à 4, pour son utilisation dans la prévention ou le traitement du diabète de type 2, du syndrome métabolique ou des troubles secondaires associés.

8. Utilisation d'au moins une fraction isolée de perméat de lactosérum doux selon l'une quelconque des revendications 1 à 4, pour la production d'un médicament destiné à la prévention ou le traitement du diabète de type 2, du syndrome métabolique ou des troubles secondaires associés.

9. Complément alimentaire, comprenant au moins l'une des fractions isolées de perméat de lactosérum doux selon l'une quelconque des revendications 1 à 4.
